(19) Europäisches Patentamt
European Patent Office
Office européen des brevets

(11) EP 0 829 475 B1

(12) **FASCICULE DE BREVET EUROPEEN**

(45) Date de publication et mention de la délivrance du brevet:
**26.07.2000 Bulletin 2000/30**

(51) Int Cl.[7]: **C07D 233/32**

(21) Numéro de dépôt: **97402064.6**

(22) Date de dépôt: **04.09.1997**

# (54) Procédé de fabrication de (méth) acrylates d'alkylimidazolidone

Verfahren zur Herstellung von Alkylimidazolidinon(meth)acrylates

Process for the preparation of alkyl imidazolidinone (meth) acrylates

(84) Etats contractants désignés:
**AT BE CH DE DK ES FI FR GB GR IE IT LI LU NL PT SE**

(30) Priorité: **16.09.1996 FR 9611270**

(43) Date de publication de la demande:
**18.03.1998 Bulletin 1998/12**

(73) Titulaire: **ELF ATOCHEM S.A.**
**92800 Puteaux, Hauts-de-Seine (FR)**

(72) Inventeurs:
- **Esch, Marc**
  **57800 Freyming Merlebach (FR)**
- **Riondel, Alain**
  **57600 Forbach (FR)**

(74) Mandataire: **Rieux, Michel et al**
**ELF ATOCHEM S.A.,**
**Département Propriété Industrielle,**
**4, Cours Michelet,**
**La Défense 10,**
**Cédex 42**
**92091 Paris la Défense (FR)**

(56) Documents cités:
**EP-A- 0 236 994**
**EP-A- 0 571 851**
**EP-A- 0 650 962**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 829 475 B1

Printed by Jouve, 75001 PARIS (FR)

## Description

La présente invention porte sur un procédé de fabrication d'un composé de formule :

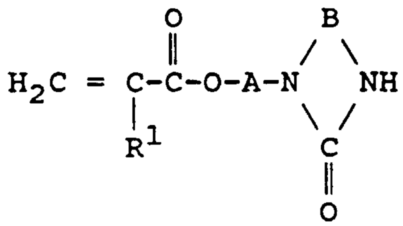

(I)

dans laquelle :

- $R^1$ représente hydrogène ou méthyle ;
- A et B représentent chacun indépendamment une chaîne hydrocarbonée droite ou ramifiée, ayant de 2 à 5 atomes de carbone,

par réaction d'au moins un (méth)acrylate de formule :

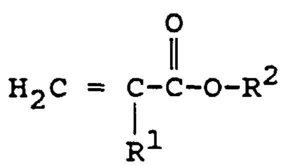

(II)

dans laquelle :

- $R^1$ a la signification précitée ; et
- $R^2$ représente un groupe alkyle en $C_1$-$C_4$,

avec un alcool hétérocyclique de formule :

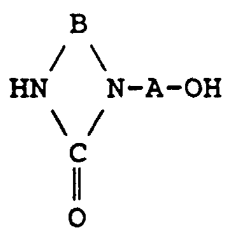

(III)

dans laquelle A et B ont les significations précitées.

Ces composés de formule (I) sont connus pour leur rôle dans la constitution de polymères utiles comme revêtements et adhésifs, pour le traitement du papier et des textiles, notamment par le brevet américain US-A-2 871 223, ainsi que pour leurs utilisations comme agents de traitement du cuir, et dans la production de peintures en émulsion. Le méthacrylate d'éthylimidazolidone (MEIO) est principalement utilisé dans les peintures en tant que promoteur d'adhésion humide.

La synthèse du MEIO par transestérification a déjà fait l'objet de très nombreux brevets, lesquels diffèrent les uns des autres par la nature du catalyseur utilisé. Des catalyseurs solides ou en partie solides, permettant d'opérer à une température inférieure à 100°C, tout en conduisant à des bons résultats du point de vue du rendement en MEIO et de la conversion de l'HEIO (1-(2-hydroxyéthyl)imidazolidyl-2-one), ont été découverts. Il apparaît toutefois que l'on pourrait rendre la synthèse encore plus facile à mettre en oeuvre à l'échelle industrielle si l'on proposait, pour cette réaction, un catalyseur liquide offrant les mêmes avantages.

Dans la demande de brevet européen EP-A-0 236 994, est signalée l'utilisation du méthylate de sodium comme catalyseur de cette réaction, mais avec la recommandation de ne pas l'utiliser en raison de sa forte propension à favoriser les réactions secondaires (addition de Michael notamment). De façon surprenante, la Société déposante a cependant maintenant découvert que ce catalyseur peut très avantageusement être utilisé, donnant de bons résultats quant au rendement en MEIO et à la conversion de l'HEIO, sans conduire à l'inconvénient annoncé de favoriser les réactions secondaires, à la condition de l'introduire non pas en une seule fois (conditions habituelles d'utilisation) au début de la synthèse, mais de façon répartie tout au long de la synthèse.

La présente invention a donc pour objet le procédé de fabrication d'un composé de formule (I), tel qu'il a été défini ci-dessus, en présence de méthylate de sodium en tant que catalyseur, ce procédé étant caractérisé par le fait que l'on introduit le méthylate de sodium en solution, de façon progressive tout au long de la synthèse.

Conformément à un mode de réalisation particulièrement préféré du procédé selon l'invention, on introduit une partie du méthylate de sodium au début de la synthèse, et le reste, en continu ou par fractions tout au long de la synthèse. La fraction que l'on introduit initialement représente avantageusement de 2 à 40% en poids, de préférence de 4 à 20% en poids, de méthylate de sodium par rapport à la quantité totale de méthylate de sodium mis en oeuvre.

On introduit généralement une solution de méthylate de sodium à 1 - 30% en poids dans un solvant tel que le méthanol.

La quantité de méthylate de sodium utilisée pour la mise en oeuvre du procédé selon l'invention est généralement comprise entre $1 \times 10^{-4}$ et $2 \times 10^{-3}$ mole, de préférence $5 \times 10^{-4}$ et $1 \times 10^{-3}$ mole, par mole de l'alcool hétérocyclique de formule (III).

Comme exemples de réactifs de formule (II), on peut citer notamment les acrylates et méthacrylates de méthyle, d'éthyle, de n-propyle, d'isopropyle, de n-butyle et d'isobutyle.

Comme exemples d'alcool hétérocyclique de formule (III), on peut citer l'HEIO.

La réaction du procédé selon l'invention peut être effectuée en présence d'un excès de l'un ou l'autre des réactifs. Il est toutefois conseillé que le rapport molaire (méth)acrylate de formule (II)/alcool hétérocyclique de formule (III) soit compris entre 2 et 5 environ, de préférence entre 2,5 et 3,5. En opérant avec un large excès molaire de (méth) acrylate par rapport à l'alcool hétérocyclique, on obtient, à l'issue de la réaction, une solution de composé de formule (I) dans le (méth)acrylate qui peut être utilisée directement pour certaines applications, telles que l'obtention de peintures et revêtements ou bien le traitement du cuir.

La réaction du procédé selon l'invention est, de préférence, effectuée en présence d'au moins un inhibiteur de polymérisation, utilisé, par exemple, à raison de 300 à 1800 ppm, de préférence de 600 à 1300 ppm, par rapport à l'alcool hétérocyclique de formule (III). Comme exemples d'inhibiteurs de polymérisation utilisables, on peut citer notamment la phénothiazine, l'éther méthylique de l'hydroquinone, le di-tert.-butyl-catéchol, l'hydroquinone, le p-anilinophénol, la paraphénylène diamine et leurs mélanges en toutes proportions.

La réaction du procédé selon l'invention est effectuée, de préférence, sous une pression ne dépassant pas la pression atmosphérique, par exemple une pression comprise entre 0,3 et 1 bar. De façon avantageuse, la réaction est réalisée sous bullage d'air pour améliorer l'efficacité des stabilisants. Elle est effectuée en mélangeant le (méth) acrylate de formule (II) et l'alcool hétérocyclique de formule (III), et en chauffant le mélange réactionnel généralement à une température comprise entre 60 et 90°C environ, de préférence entre 70 et 85°C environ, cette température étant évidemment dépendante de la nature exacte de l'alcool et du (méth)acrylate.

Dans la mise en oeuvre du procédé selon l'invention, il est conseillé d'atteindre une déshydratation maximale avant l'addition du catalyseur, de manière à éviter la désactivation de ce dernier par l'eau. On peut parvenir à ce résultat, par exemple, en chauffant le mélange initial de (méth)acrylate de formule (II), d'alcool hétérocyclique de formule (III) et d'inhibiteurs de polymérisation au reflux, tout en séparant par distillation l'azéotrope de (méth)acrylate et d'eau lorsqu'il se forme un azéotrope de (méth)acrylate et d'eau. A ce stade, après séparation du distillat, la fraction initiale de catalyseur est introduite dans le mélange réactionnel à chaud.

La durée de la réaction selon l'invention dépend évidemment de conditions réactionnelles, telles que la température, la pression et la quantité de catalyseur utilisée, mais est généralement comprise entre 5 et 10 heures environ. Elle dépend évidemment aussi de la nature des réactifs mis en oeuvre.

Le mélange réactionnel est donc chauffé au reflux jusqu'à ce que la température de tête atteigne la température de distillation de l'azéotrope du (méth)acrylate et de l'alcool de formule $R_2OH$ formé par la réaction lorsqu'il se forme un azéotrope.

L'excès éventuel de (méth)acrylate peut ensuite être éliminé par évaporation, de manière à isoler le composé de formule (I) du milieu réactionnel, généralement à l'état solide : ainsi, l'acrylate de 1-(2-hydroxyéthyl)imidazolidyl-2-one est un solide blanc cristallin de température de fusion égale à 43'C, soluble à froid dans les cétones, les alcools, les hydrocarbures aromatiques et l'eau, insolubles à froid dans les hydrocarbures saturés et qui précipite à 0°C dans l'acrylate d'éthyle. Le méthacrylate de 1-(2-hydroxyéthyl)imidazolidyl-2-one est un solide blanc cristallin de température de fusion égale à 47°C, possédant les mêmes propriétés de solubilité que l'acrylate précédent. A l'issu de l'opération d'évaporation, le produit solide cristallin peut en outre être purifié par filtration, puis lavage à l'aide d'éther de pétrole,

et séchage.

L'isolement du composé (I) peut aussi être réalisé par évaporation partielle du (méth)acrylate, puis cristallisation à une température suffisamment basse (de préférence inférieure ou égale à 0°C) et pendant une durée suffisamment longue (pouvant atteindre jusqu'à 15 heures), puis filtration suivie des étapes de purification décrites ci-dessus.

Enfin, une troisième méthode pour isoler le composé de formule (I) de la solution le contenant, consiste à effectuer une extraction par l'eau, suivie d'une décantation, d'une concentration du (méth)acrylate et des étapes de purification décrites ci-dessus.

Les Exemples suivants illustrent l'invention sans toutefois la limiter. Dans ces Exemples, les pourcentages sont indiqués en poids sauf indication contraire.

EXEMPLE 1

Dans un réacteur en verre, à double enveloppe, de 5 litres, équipé d'une sonde de mesure de la température, d'un agitateur mécanique à vitesse variable, d'une colonne adiabatique à garnissage surmontée d'une tête de reflux, on introduit 1458 g d'HEIO, 1,823 g de phénothiazine (PTZ) en tant que stabilisant et 3827 g de méthacrylate de méthyle (MAM). On met en service une stabilisation en tête de colonne par une solution à 0,1% d'éther méthylique de l'hydroquinone (EMHQ) dans le MAM.

Sous bullage d'air et sous pression réduite, on porte le contenu du réacteur à ébullition, à une température de 75'C, pendant 1 heure, et on élimine l'eau par distillation azéotropique avec le MAM.

Ensuite, on introduit en une seule fois dans le réacteur 2,12 g de méthylate de sodium (MeONa) à 1% dans le méthanol (MeOH). Le restant du MeONa à 1% dans le MeOH, soit 40,82 g, est introduit en 7 heures à l'aide d'une pompe doseuse. On ajuste la pression pour maintenir dans le réacteur une température de 75'C. On régule le soutirage de l'azéotrope MAM-MeOH par une température de consigne en tête de colonne égale à celle de l'ébullition de l'azéotrope sous la pression considérée plus 3'C. Lorsque la quantité du méthanol soutirée correspond à la quantité attendue, on prolonge la réaction d'une heure, jusqu'à ce que l'on ne constate plus de formation de MeOH (température en tête de colonne mesurée à reflux total = température d'ébullition du MAM sous la pression considérée).

Après refroidissement, on récupère du MEIO brut, exempt de solide.

Le rendement en MEIO et la conversion de 1'HEIO sont déterminés d'après l'analyse par chromatographie en phase liquide (HPLC) du brut réactionnel, par les équations suivantes :

$$\text{Conversion de l'HEIO C (\%)} = \frac{\text{(HEIO de départ - HEIO final)}}{\text{HEIO de départ}} \times 100$$

$$\text{Rendement en MEIO R (\%)} = \frac{\text{Nombre de moles de MEIO formé}}{\text{Nombre de moles d'HEIO de départ}} \times 100$$

Les résultats sont rapportés dans le Tableau ci-après, lequel comprend également les données et résultats de l'Exemple Comparatif 2.

EXEMPLE COMPARATIF 2

On reproduit le même essai qu'à l'Exemple 1, excepté que la totalité du catalyseur MeONa est introduite en une seule fois au début de la synthèse.

Tableau 1

| Exemple | Analyse HPLC du mélange brut obtenu (%) | | | R (%) | C (%) |
|---|---|---|---|---|---|
| | MAM | HEIO | MEIO | | |
| 1 (de l'invention) | 43,58 | 1,44 | 41,66 | 77 | 95,9 |
| 2 (comparatif) | Non analysé : le mélange est biphasique, synonyme d'une conversion inférieure à 50% | | | | <50 |

**Revendications**

1. Procédé de fabrication d'un composé de formule :

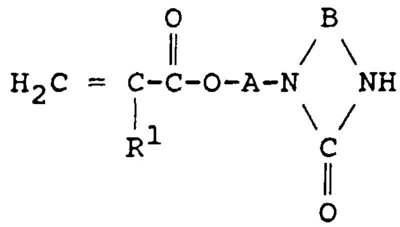 (I)

dans laquelle :

- $R^1$ représente hydrogène ou méthyle ;
- A et B représentent chacun indépendamment une chaîne hydrocarbonée droite ou ramifiée, ayant de 2 à 5 atomes de carbone,

par réaction d'au moins un (méth)acrylate de formule :

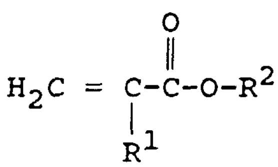 (II)

dans laquelle :

- $R^1$ a la signification précitée ; et
- $R^2$ représente un groupe alkyle en $C_1$-$C_4$,

avec un alcool hétérocyclique de formule :

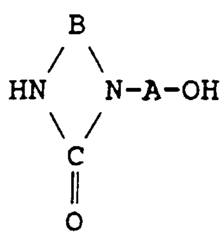 (III)

dans laquelle A et B ont les significations précitées, en présence de méthylate de sodium comme catalyseur, caractérisé par le fait que l'on introduit le méthylate de sodium en solution, de façon progressive tout au long de la synthèse.

**2.** Procédé selon la revendication 1, caractérisé par le fait que l'on introduit une partie du méthylate de sodium au début de la synthèse, et le reste, en continu ou par fractions tout au long de la synthèse.

**3.** Procédé selon la revendication 2, caractérisé par le fait que l'on introduit initialement 2 à 40% en poids, de préférence 4 à 20% en poids, de méthylate de sodium par rapport à la quantité totale de méthylate de sodium mise en oeuvre.

**4.** Procédé selon l'une des revendications 1 à 3, caractérisé par le fait que l'on introduit une solution de méthylate de sodium à 1-30% en poids dans un solvant tel que le méthanol.

5. Procédé selon l'une des revendications 1 à 4, caractérisé par le fait que l'on utilise le méthylate de sodium dans une quantité de $1 \times 10^{-4}$ à $2 \times 10^{-3}$ mole, de préférence de $5 \times 10^{-4}$ à $1 \times 10^{-3}$ mole, par mole de l'alcool hétérocyclique de formule (III).

6. Procédé selon l'une des revendications 1 à 5, caractérisé par le fait que l'on conduit la réaction à une température comprise entre 60 et 90°C, de préférence entre 70 et 85°C.

7. Procédé selon l'une des revendications 1 à 6, caractérisé par le fait que l'on utilise un rapport molaire du (méth) acrylate de formule (II) à l'alcool hétérocyclique de formule (III) qui est compris entre 2 et 5, de préférence entre 2,5 et 3,5.

8. Procédé selon l'une des revendications 1 à 7, caractérisé par le fait que l'on conduit la réaction pendant une durée comprise entre 5 et 10 heures, sous une pression ne dépassant pas la pression atmosphérique.

9. Procédé selon l'une des revendications 1 à 8, caractérisé par le fait que l'on conduit la réaction en présence d'au moins un inhibiteur de polymérisation choisi parmi la phénothiazine, l'éther méthylique de l'hydroquinone, le ditertiobutylcatéchol, l'hydroquinone, le p-anilinophénol, la paraphénylène diamine et leurs mélanges en toutes proportions.

10. Procédé selon la revendication 9, caractérisé par le fait que l'on utilise le ou les inhibiteurs de polymérisation à raison de 300 à 1800 ppm, de préférence de 600 à 1300 ppm, par rapport à l'alcool hétérocyclique de formule (III).

**Patentansprüche**

1. Verfahren zur Herstellung einer Verbindung der Formel

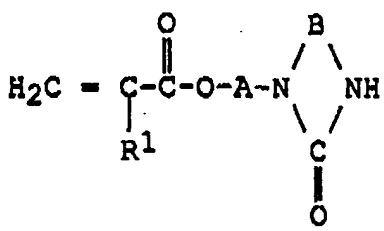

(I)

in der

- $R^1$ Wasserstoff oder Methyl bedeutet;
- A und B jeweils unabhängig voneinander eine geradkettige oder verzweigte Kohlenwasserstoffkette mit 2 bis 5 Kohlenstoffatomen bedeuten,

durch Umsetzung mindestens eines (Meth)acrylats der Formel:

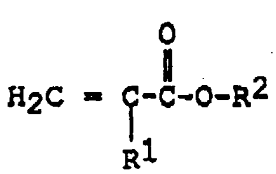

(II)

in der

- $R^1$ die vorstehend erwähnte Bedeutung hat; und
- $R^2$ eine $C_1$-$C_4$-Alkylgruppe bedeutet,

mit einem heterocyclischen Alkohol der Formel:

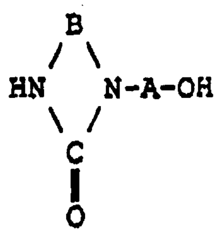

(III)

in der

A und B die vorerwähnten Bedeutungen haben, in Gegenwart von Natriummethylat als Katalysator, dadurch gekennzeichnet, daß man das Natriummethylat in Lösung nach und nach während der gesamten Herstellung zusetzt.

**2.** Verfahren nach Anspruch 1, dadurch gekennzeichnet, daß man einen Teil des Natriummathylats zu Beginn der Herstellung zuführt und den Rest kontinuierlich oder in Teilmengen während der gesamten Herstellung zugibt.

**3.** Verfahren nach Anspruch 2, dadurch gekennzeichnet, daß man zu Beginn 2 bis 40 Gew.-% und vorzugsweise 4 bis 20 Gew.-% des Natriummethylats, bezogen auf die Gesamtmenge des eingesetzten Natriumnethylats, zugibt.

**4.** Verfahren nach einem der Ansprüche 1 bis 3, dadurch gekennzeichnet, daß man eine Lösung von Natriummethylat mit einem Gehalt an 1-30 Gew.-% in einem Lösungsmittel, wie Methanol, zugibt.

**5.** Verfahren nach einem der Ansprüche 1 bis 4, dadurch gekennzeichnet, daß man das Natriummethylat in einer Menge von $1 \times 10^{-4}$ bis $2 \times 10^{-3}$ Mol und vorzugsweise von $5 \times 10^{-4}$ bis $1 \times 10^{-3}$ Mol pro 1 Mol des heterocyclischen Alkohols der Formel (III) verwendet.

**6.** Verfahren nach einem der Ansprüche 1 bis 5, dadurch gekennzeichnet, daß man die Umsetzung bei einer Temperatur von 60 bis 90°C und vorzugsweise von 70 bis 85°C durchführt.

**7.** Verfahren nach einem der Ansprüche 1 bis 6, dadurch gekennzeichnet, daß man ein Molverhältnis von (Meth) acrylat der Formel (II) zum heterocyclischen Alkohol der Formel (III) anwendet, das im Bereich von 2 bis 5 und vorzugsweise von 2,5 bis 3,5 liegt.

**8.** Verfahren nach einem der Ansprüche 1 bis 7, dadurch gekennzeichnet, daß man die Umsetzung für eine Dauer von 5 bis 10 Stunden unter einem Druck, der den atmosphärischen Druck nicht übersteigt, durchführt.

**9.** Verfahren nach einem der Ansprüche 1 bis 8, dadurch gekennzeichnet, daß man die Umsetzung in Gegenwart von mindestens einem Polymerisationsinhibitor durchführt, der unter Phenothiazin, Hydrochinonmethylether, Ditert.-butylcatechin, Hydrochinon, p-Anilinophenol, p-Phenylendiamin und Gemischen dieser Verbindungen in beliebigen Verhältnissen ausgewählt ist.

**10.** Verfahren nach Anspruch 9, dadurch gekennzeichnet, daß man den oder die Polymerisationsinhibitoren in einem Anteil von 300 bis 1800 ppm und vorzugsweise von 600 bis 1300 ppm, bezogen auf den heterocyclischen Alkohol der Formel (III) verwendet.

## Claims

**1.** Process for the manufacture of a compound of formula:

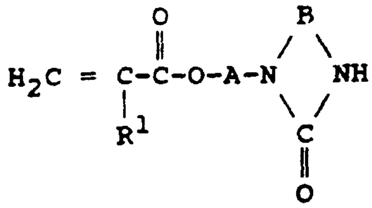

(I)

in which:

- $R^1$ represents hydrogen or methyl;
- A and B each independently represent a straight or branched hydrocarbon chain having from 2 to 5 carbon atoms,

by reaction of at least one (meth)acrylate of formula:

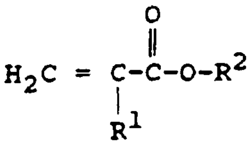

(II)

in which:

- $R^1$ has the abovementioned meaning; and
- $R^2$ represents a $C_1$-$C_4$ alkyl group,

with a heterocyclic alcohol of formula:

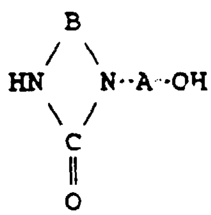

(III)

in which A and B have the abovementioned meanings,
in the presence of sodium methoxide as catalyst,
characterized in that the sodium methoxide is gradually introduced, as a solution, throughout the synthesis.

2. Process according to Claim 1, characterized in that a portion of the sodium methoxide is introduced at the beginning of the synthesis and the remainder is introduced, continuously or in fractions, throughout the synthesis.

3. Process according to Claim 2, characterized in that 2 to 40% by weight, preferably 4 to 20% by weight, of sodium methoxide are introduced initially with respect to the total amount of sodium methoxide employed.

4. Process according to one of Claims 1 to 3, characterized in that a 1-30% by weight solution of sodium methoxide in a solvent, such as methanol, is introduced.

5. Process according to one of Claims 1 to 4, characterized in that the sodium methoxide is used in an amount of $1 \times 10^{-4}$ to $2 \times 10^{-3}$ mol, preferably of $5 \times 10^{-4}$ to $1 \times 10^{-3}$ mol, per mole of the heterocyclic alcohol of formula (III).

6. Process according to one of Claims 1 to 5, characterized in that the reaction is carried out at a temperature of

between 60 and 90°C, preferably between 70 and 85°C.

**7.** Process according to one of Claims 1 to 6, characterized in that a molar ratio of the (meth)acrylate of formula (II) to the heterocyclic alcohol of formula (III) is used which is between 2 and 5, preferably between 2.5 and 3.5.

**8.** Process according to one of Claims 1 to 7, characterized in that the reaction is carried out for a period of time of between 5 and 10 hours at a pressure not exceeding atmospheric pressure.

**9.** Process according to one of Claims 1 to 8, characterized in that the reaction is carried out in the presence of at least one polymerization inhibitor chosen from phenothiazine, hydroquinone methyl ether, di-tert-butylcatechol, hydroquinone, p-anilinophenol, para-phenylenediamine and their mixtures in all proportions.

**10.** Process according to Claim 9, characterized in that the polymerization inhibitor or inhibitors is or are used in the proportion of 300 to 1800 ppm, preferably of 600 to 1300 ppm, with respect to the heterocyclic alcohol of formula (III).